# EUROPEAN PATENT SPECIFICATION

(11) **EP 2 742 070 B1**
(45) Date of publication and mention of the grant of the patent: **15.09.2021**
(21) Application number: 12755897.1
(22) Date of filing: 31.07.2012
(51) Int. Cl.: C08B 37/00, C08L 5/08, A61K 8/73, A61K 47/36, A61L 27/52, C08J 3/075, A61K 9/00, A61K 31/728

(54) **DEGRADATION-RESISTANT CROSS-LINKED, LOW-MOLECULAR-WEIGHT HYALURONATE**
ABBAU-STABILE, VERNETZTE, NIEDERMOLEKULARE HYALURONSÄURE
ACIDE HYALURONIQUE RÉTICULÉ À FAIBLE POIDS MOLÉCULAIRE RÉSISTANT À LA DÉGRADATION

(30) Priority: 10.08.2011 EP 11006559
(43) Date of publication of application: 18.06.2014
(73) Proprietor: Glycores 2000 srl, 20155 Milan (IT)
(72) Inventor: ORESTE, Pasqua, Anna, I-20155 Milan (IT)
(74) Representative: Trupiano, Federica
(86) International application number: PCT/IB2012/001472
(87) International publication number: WO 2013/021249

(56) References cited:
- WO-A1-90/09401
- WO-A1-2005/066215
- WO-A1-2008/000260
- WO-A1-2008/126803
- WO-A1-2010/043106
- WO-A2-2010/029344
- WO-A2-2011/023355
- GB-A- 2 401 043
- KR-A- 20080 062 092
- KR-A- 20080 073 419
- US-A- 4 963 666
- US-A1- 2004 127 698
- JUNGJU KIM ET AL: "Synthesis and characterization of matrix metalloprotease sensitive-low molecular weight hyaluronic acid based hydrogels", JOURNAL OF MATERIALS SCIENCE: MATERIALS IN MEDICINE, KLUWER ACADEMIC PUBLISHERS, BO, vol. 19, no. 11, 22 May 2008 (2008-05-22), pages 3311-3318, XP019607927, ISSN: 1573-4838 cited in the application
- BARBUCCI R ET AL: "Hyaluronic acid hydrogel in the treatment of osteoarthritis", BIOMATERIALS, ELSEVIER SCIENCE PUBLISHERS BV., BARKING, GB, vol. 23, no. 23, 1 December 2002 (2002-12-01), pages 4503-4513, XP004377521, ISSN: 0142-9612, DOI: 10.1016/S0142-9612(02)00194-1
- GIAVARESI G ET AL: "Blood vessel formation after soft-tissue implantation of hyaluronan-based hydrogel supplemented with copper ions", BIOMATERIALS, ELSEVIER SCIENCE PUBLISHERS BV., BARKING, GB, vol. 26, no. 16, 1 June 2005 (2005-06-01), pages 3001-3008, XP025280643, ISSN: 0142-9612, DOI: 10.1016/J.BIOMATERIALS.2004.08.027 [retrieved on 2005-06-01]
- XIAO ZHENG SHU ET AL: "Disulfide Cross-Linked Hyaluronan Hydrogels", BIOMACROMOLECULES, vol. 3, no. 6, 1 November 2002 (2002-11-01), pages 1304-1311, XP055005444, ISSN: 1525-7797, DOI: 10.1021/bm025603c
- Anonymous: "1,4-Butanediol diglycidyl ether >=95% | Sigma-Aldrich", , 1 January 2019 (2019-01-01), XP055580145, Retrieved from the Internet: URL:https://www.sigmaaldrich.com/catalog/p roduct/aldrich/220892?lang=en ion=NL [retrieved on 2019-04-11]
- Anonymous: "Gel - Wikipedia, the free encyclopedia", , 30 June 2015 (2015-06-30), XP055219020, Retrieved from the Internet: URL:https://en.wikipedia.org/wiki/Gel [retrieved on 2015-10-07]
- George A Paleos: "What are Hydrogels?", , 1 January 2012 (2012-01-01), XP055324484, Retrieved from the Internet: URL:http://pittsburghplastics.com/assets/f iles/What Are Hydrogels.pdf [retrieved on 2016-11-30]
- REDDY NARENDRA ET AL: "Crosslinking biopolymers for biomedical applications", TRENDS IN BIOTECHNOLOGY, vol. 33, no. 6, June 2015 (2015-06), pages 362-369, XP029173056, ISSN: 0167-7799, DOI: 10.1016/J.TIBTECH.2015.03.008
- O. Okay: "General Properties of Hydrogels" In: "Springer Series on Chemical Sensors and Biosensors", 1 January 2009 (2009-01-01), Springer, Germany, XP055324490, ISSN: 1612-7617 vol. 6, pages 1-14, DOI: 10.1007/978-3-540-75645-3_1,
- ELBERT ET AL: "Liquid-liquid two-phase systems for the production of porous hydrogels and hydrogel microspheres for biomedical applications: A tutorial review", ACTA BIOMATERIALIA, ELSEVIER, AMSTERDAM, NL, vol. 7, no. 1 1 January 2011 (2011-01-01), pages 31-56, XP027448746, ISSN: 1742-7061, DOI: 10.1016/J.ACTBIO.2010.07.028 [retrieved on 2010-07-24]
- N G Nanjundswamy: "A Review on Hydrogels and Its Use in In Situ Ocular Drug Delivery", Indian Journal of Novel Drug Delivery, 1 January 2009 (2009-01-01), pages 11-17, XP055324499,
- Executive Summary: Dermal Filler Devices

## Description

### OBJECT OF THE INVENTION

The present invention relates to a biodegradable but degradation-resistant product obtained by modification of low molecular weight hyaluronic acid for use in biomedical, pharmaceutical and cosmetic applications. More particularly, the invention provides a process for the preparation of a cross-linked, low-molecular-weight hyaluronic acid hydrogel having a molecular weight of from 50,000 to 100,000, a higher resistance to enzymatic degradation than the commercial, cross-linked hyaluronates with claimed long term biodegradation kinetics, and morphologic characteristics which are greatly improved in respect of the heretofore described cross-linked hyaluronates. The invention also provides the cross-linked, low-molecular-weight hyaluronic acid hydrogel obtained by the process of the invention.

Said low-molecular-weight hyaluronic acid, cross-linked *via* the 1,4-butanediol-diglycidylether (BDDE) cross-linking agent, is a solid (semisolid) but viscoelastic, transparent hydrogel which can be easily employed as viscosupplementation filler for treating joint diseases or as filler for tissue relaxation treatment (wrinkles) in cosmetics.

### BACKGROUND OF THE INVENTION

Hyaluronic acid (HA) is a glycosaminoglycan which is present in the hyaline cartilage, synovial joint fluid and skin tissues. More particularly, HA is a linear glycosaminoglycan formed by a mixture of chains of different length constituted by the repetition of a regular disaccharide formed by a glucuronic acid unit and a N-acetyl-glucosamine unit linked *beta* 1-4. Disaccharides are linked *beta* 1-3 with an average molecular weight up to 6 Md (6x10⁶ Da). Therefore, each chain in said mixture of chains shows the same repetitive sequence of formula (A) 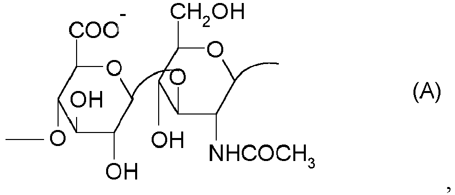 the corresponding cation generally being hydrogen (hyaluronic acid) or sodium (sodium hyaluronate).

In the tissues, the function of hyaluronic acid is mainly to maintain the structural density allowing in the same time the biochemical actions of the natural products in the specific body districts. In fluids like synovia the action of HA is to keep the right viscosity by a lubricant action. To exert these actions, HA needs to be fully biocompatible including a right metabolic balance. Natural HA is continuously degraded and synthesized by the body enzymes. This homeostasis is deviated when pathological situations occur, therefore increases in the HA catabolism can results in wide range of effects from a severe pathology to simple tissue modifications. The application of HA, as sodium hyaluronate, as filler in cosmetic or in viscoelastic replacement in synovitis, requires that the employed HA polymer has enhanced viscoelastic properties. This rheology has to be balanced with an efficient capability to make the production of the injectable product.

The industrial hyaluronic acid is obtained by extraction from animal tissues or by microorganism fermentation and is commonly available as sodium hyaluronate. Concerning molecular weight, it is generally recognized that low molecular weight HA is a mixture of chains having a mean molecular weight below 250 Kd (2.5x10⁵ Da). HA is used, generally as sodium hyaluronate, in many applications in cosmetics, ophthalmology, rheumatology and tissues engineering. In particular HA with a mean molecular weight above 1 Md is used as viscosupplement in joint arthrosis or in wrinkle management. The high molecular weight is required to supplement the synovial fluid or to fill skin connective dead spaces thanks to the viscosity of the resulting solution.

Many medicaments based on the above technology are currently available on the market. They have a high biocompatibility but they are subjected to a rather rapid degradation by the body enzymes, in particular by hyaluronidase, with the consequence of a short half-life.

### DESCRIPTION OF THE PRIOR ART

According to literature, the HA half-life has been increased by modifying HA by chemical reactions, in particular by cross-linking the HA chains each other by means of divalent reagents. Such divalent agents, well known to the persons working in this area, generally are bi- or polyfunctional epoxides, e.g. lower aliphatic epoxides, or their corresponding halohydrins or epihalohydrins or halides, forming ether bridges to the hyaluronic acid molecules. As suitable examples may be mentioned epichlorohydrin, divinylsulfone, 1,4-butanediol diglycidyl ether (BDDE), 1,2-ethylenediol diglycidyl ether, 1-(2,3-epoxypropyl)-2,3-epoxy cyclohexane, N,N-diglycidyl aniline and epoxy-substituted pentaerythritol. Other crosslinking agents that may be employed are reactive polymers such as for instance dextran or starch which have been reacted to contain e.g. glycidyl ether groups, as described in EP 272300 (WO87/78989) also disclosing the cross-linking *via* BDDE.

U.S. Patent n. 4,582,865 and WO 2006/056204 describe the preparation of cross-linked HA, alone or in combination with hydrophilic polymers from divinyl sulfone (DVS), generating a 1,2-ethylene cross-linkage.

Polyfunctional epoxy compounds have been described in EP 0161887. Formaldehyde (US 4,713,448), generating a methylene cross-linkage and carbodiimides (US 5,017,229 and US 6,013,679), generating a carbonyl cross-linkage, have been described as cross-linking agents.

A one-step method for cross-linking HA with divalent epoxy reagent and the product obtained using a high concentration of HA has been reported in US 2005/0281880. All these documents describe the use of HA with mean molecular weight above 500 Kd as starting material.

The modification of HA by chemical reactions, and in particular in the cross-linking process decreases the binding property of HA to the natural degrading enzymes, in particular to hyaluronidase. When HA is cross-linked, a tridimensional network is formed and the polysaccharide chains free energy is reduced. As lower is the molar ratio HA/cross-linking reagent applied as higher is the resistance to the degradation by the natural enzyme hyaluronidase. For example, according to the above-mentioned US 2005/0281880, the rate of enzymatic degradation is reduced from 60-90% to 10% by acting on the method of production working with water solution concentrations of HA up to 20% and an HA/cross-linking reagent molar ratio from 1:1 to 1:2.4.

US 4,716,154 discloses a substitute for vitreous humor to be used in ophthalmology, consisting of a gel of cross-linked hyaluronic acid and also states that the molecular weight of the hyaluronic acid is not a critical factor for the practice of the invention claimed therein. According to this document, HA substrates having a mean molecular weight of from 500,000 to 3,000,000 Da are preferred, but a cross-linked HA wherein the substrate has a mean molecular weight of 20,000 is described.

US 2005/0281880 (Wang) discloses a processes for preparation of injectable hyaluronan hydrogels from a hyaluronan substrate having a mean molecular weight of 2.3 MDa, said process including the steps of crosslinking one or more polymers and washing the subsequently formed gel, followed by purification and homogenization to produce an injectable hydrogel. This document specifically indicates the parameters to be used to obtain a hydrogel from hyaluronic acid.

The presently available fillers for viscosupplementation, based on native, biotechnological or cross-linked hyaluronic acid, have not a fully satisfactory therapeutic efficacy. This is due not only to the decreased hyaluronidase action but also to the whole HA catabolism in which many natural factors are involved. By consequence, the HA administration frequency is increased in function of the specific application.

Thus, there is a need of a product, for use as a viscosupplementation filler with enhanced capabilities to retain the high viscous properties and less affinity for hyaluronidase, thus being capable of prolonging the half-life of HA especially in the case of its dermal, intradermal or intra-articular use, but it is important that the product retains the characteristic to be depolymerized by the endogenous enzymes in order to avoid toxicity.

### SUMMARY OF THE INVENTION

Considering the results of the above-cited previously published studies, in particular the fact that the HA starting material for the production of cross-linked products for use as viscosupplemetation fillers are polymers with a mean molecular weight higher than 500 Kd, i.e. linear polymer chains accounting a minimum of 1250 disaccharides, we calculated that, taking into account an efficiency reaction of 5-10% using a cross-linking reagent, statistically every chain should bear 12 substitutions, thus leaving about 100-200 disaccharides unreacted. Thus, we assumed that a cross-linked product coming from a HA substrate with a reduced chains' length would be less inclined to an enzymatic degradation, especially by hyaluronidases. We calculated that cross-linked product using a starting HA of 100 Kd, the statistical unmodified chain length would be reduced to 10-20 disaccharides, with a resulting potential capability of decreasing the degradation kinetic. Cross-linked products obtainable with HA with mean MW below 50,000 could be synthesized, but to obtain a suitable gel BDDE agent has to be increased. Considering the above mentioned accessible chain calculation of 100-200 disaccharides in the case of 1,000 KDa HA sample and 10-20 in the case of the 100 KD HA sample, the cross-linking product using a HA sample of less than 50 KDa will results in an accessible chain of less than 5-10 disaccharide. This would be an unsuitable compound to keep a minimal biodegradation in vivo.

However, the purpose of obtaining a cross-linked HA with a molecular weight of from 50,000 to 100,000 faced the problem of the objective difficulty in obtaining a product having the morphologic characteristics suitable for a use in humans, by means of a safe and easy process.

In fact, notwithstanding several citations of cross-linked, low-molecular-weight hyaluronates, literature does not disclose solid but viscoelastic and transparent cross-linked, low-molecular-weight HAs hydrogels from a hyaluronate of 50,000-100,000 Da for use as viscosupplementation fillers in humans to be used also in injectable forms.

For example, WO 2005/066215 discloses Ultra-Low MW Polymer-Based Gels Solutions of various ultra-low MW HA ( < 500 KDa), including a solution of HA 60 KDa, but it does not describe any solid and transparent gel of a cross-linked HA of 60,000 Da.

Junju Kim et al. (J Materials Science: Materials in Medicine, 2008, 19/11, 3311-3318) disclose a cross-linked hyaluronic acid 50,000 Da, prepared from an acrylated HA using a polypeptide as cross-linking agent, as an hydrogel for scientific experiments, but they do not describe any viscoelastic, solid and transparent non-derivatized, cross-linked HA hydrogel of 50,000 Da suitable as supplementation filler.

GB 2401043 discloses the preparation of a degradable gel by cross-linking hyaluronic acid together with a drug, including the cross-linking of a 90 KDa HA using ethylene glycol diglycidyl ether as cross-linking agent, in the presence of danazol, but it does not describe any viscoelastic, solid and transparent cross-linked HA hydrogel of 90,000 Da suitable as supplementation filler. On the contrary, this document emphasized the degradability of the system just in view of its use as a carrier of a drug. WO 2010/029344 discloses the preparation of a cryogel from hyaluronic acid of different molecular weight, including a 50,000 Da substrate, cross-linked with 3-glycidoxypropyltrimethoxysilane, and a 100,000 Da substrate, cross-linked with glycidyl methacrylate, N,N-methylene-bis-acrylamide and N,N,N',N'-tetrametylenedinamine by using the classical method used to make the polyacrylamide gel for the electrophoresis, but said document does not disclose any viscoelastic, solid and transparent cross-linked HA hydrogel of 50,000 Da or 100,000 Da. On the contrary, the obtained product is a spongy cryogel.

WO 90/09401 discloses a method for crosslinking hyaluronic acid by subjecting a solution of hyaluronic acid by using a phosphorus-containing reagent, and the preparation of a sodium hyaluronate 100,000 Da cross-linked with POCl₃, i.e. with a potentially dangerous explosive agent, but the document does not describe any viscoelastic, solid and transparent cross-linked HA hydrogel of 100,000 Da suitable as supplementation filler, because the obtained product is an opalescent brittle gel. According to this document, the gels obtained according to the above-mentioned EP 272300, disclosing the cross-linking *via* BDDE, cannot be prepared in an injectable form.

By applying the process of the aforementioned US 2005/0281880, i.e. by reacting sodium hyaluronate (Na-HA) with BDDE under the conditions described therein, no hydrogel is obtained because either no cross-linked product forms or the possibly formed product remains in solution.

Actually, the literature does not disclose viscoelastic, semisolid and transparent (clear) cross-linked low-molecular weight (50,000-100,000 kDa) HA hydrogels.

Now, it has been found that, by applying the cross-linking reaction to low molecular weight sodium hyaluronate, but not to very low molecular weight sodium hyaluronate in order to prolong the half-life of the compound avoiding any toxicity in particular by using a sodium hyaluronate substrate having a mean molecular weight from 50,000 Da to 100,000 Da, by using 1,4-butanediol diglycidyl ether (BDDE) as cross-linking agent under precise conditions, a viscoelastic, semisolid and transparent (clear) cross-linked sodium hyaluronate hydrogel is obtained which is very resistant to the degradation by bacterial hyaluronidase.

In fact, it has been found that, by carrying out the cross-linking reaction under particular conditions, i.e. by using a sodium hyaluronate substrate of mean molecular weight of 100.000 Da in 1% sodium hydroxide at a concentration of from 18% to 22% and the cross-linking agent in a HA-Na/Cross-linking agent molar ratio from 1:55 to 1:60 it is possible to obtain a hydrogel for use for viscosupplementation having a resistance to the degradation by hyaluronidase much higher than that of a high molecular weight cross-linked sodium hyaluronate, thus confirming the hypothesis that the present inventors illustrate in the Background of the Invention.

In addition, it has been found that it is possible to obtain viscoelastic, semisolid and transparent, low-molecular-weight cross-linked hyaluronates hydrogels by reacting an 8-40% solution of a sodium hyaluronate having a mean molecular weight of from 50,000 to 100,000 Da in a 0.75%-1.5% aqueous solution of sodium hydroxide with an amount of 1,4-butanediol diglycidyl ether corresponding to from 180 moles to 500 moles per mole of the sodium hyaluronate.

Surprisingly, it has also been found that the BDDE cross-linked HAs of from 50,000 Da to 100,000 Da thus obtained are not spongy or anyway highly porous as those described in the literature, in particular as the corresponding BDDE cross-linked native hyaluronan described by G.D Prestwich in the review "Biomaterials from Chemically-Modified Hyaluronan" Glycoforum.gr.jp/science/hyaluronan/HA18/HA18E.html, but are viscoelastic, semisolid and clear products which are particularly adapted as viscosupplementation fillers.

### BRIEF DESCRIPTION OF THE DRAWINGS

- Figure 1 illustrates the enzyme kinetics of the 1,000 KDa cross-linked sodium hyaluronate hydrogel of PREPARATION 1 compared with that of native sodium hyaluronate.
- Figure 2 illustrates the enzyme kinetics of the crosslinked sodium hyaluronate 100 KDa hydrogel of Example 1 compared with that of with native sodium hyaluronate 1,000 KDa and Native sodium hyaluronate 100 KDa.
- Figure 3 illustrates the enzyme kinetics of the cross-linked sodium hyaluronate 100 KDa hydrogel of Example 1 compared with that of a commercial cross-linked sodium hyaluronate.
- Figure 4 illustrates the enzyme kinetics of the native sodium hyaluronate 100 KDa and of the cross-linked sodium hyaluronate 100 KDa hydrogels of Examples 1-3
- Figure 5 illustrates the enzyme kinetics of the native sodium hyaluronate 50 KDa and of the cross-linked sodium hyaluronate 50 KDa hydrogels of Examples 5-6 and of the cross-linked sodium hyaluronate 100 KDa hydrogel of Example 1.

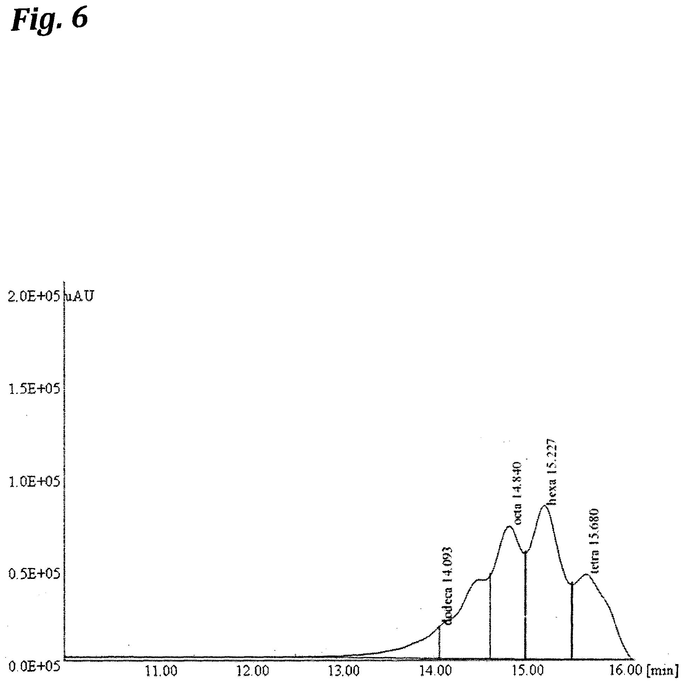

- Figure 6 illustrates the GPC-HPLC profile of Hyaluronic acid with 1,000 Kd molecular weight depolymerized with mammalian hyaluronidase after 7 hours of reaction. Signals of dodeca (14.09 min), octa (14.84 min), hexa (15.22 min) and tetrasaccharides (15.68) are indicated in the chromatogram.

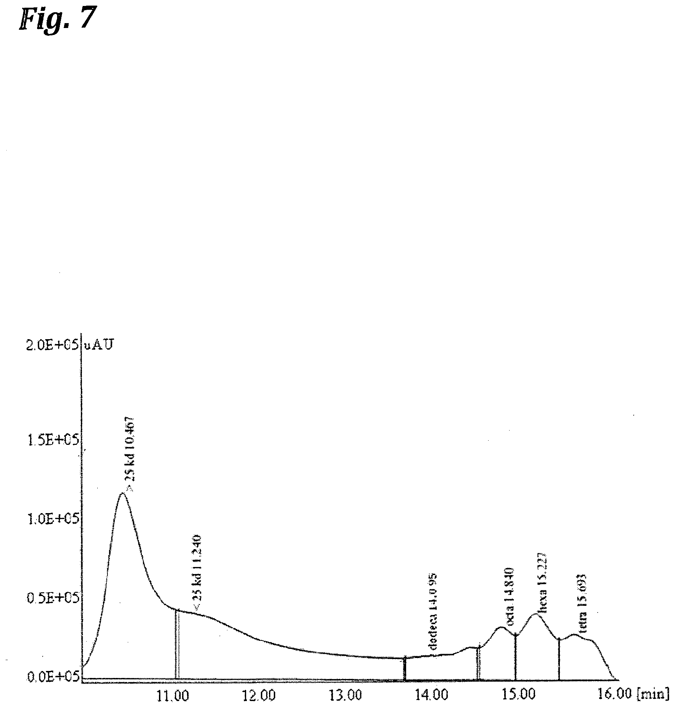

- Figure 7 illustrates the GPC-HPLC profile of a commercial cross-linked product (Durolane) depolymerized with mammalian hyaluronidase after 7 hours of reaction. Signals of high molecular weight oligomers above (10.46 min) and below 25,000 Da (11.24 min) and signals of dodeca (14.09 min), octa (14.84 min), hexa (15.22 min) and tetrasaccharides (15.69) are indicated in the chromatogram.

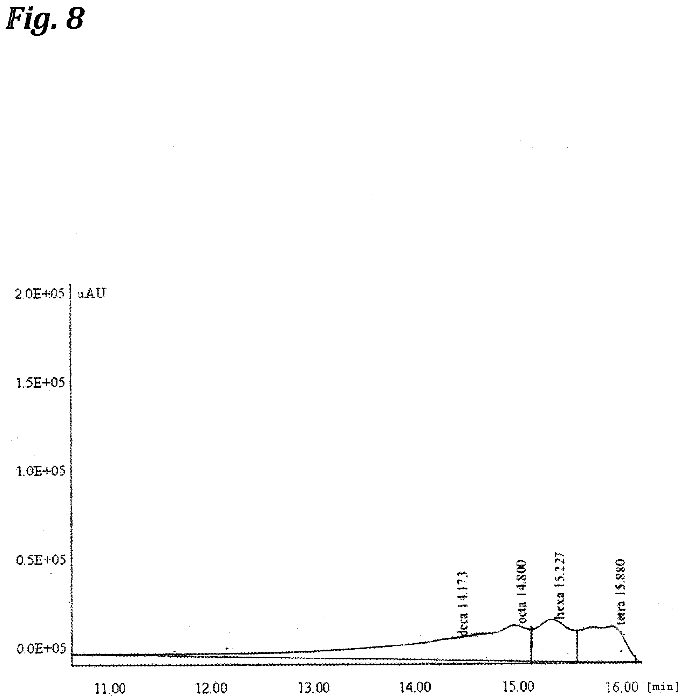

- Figure 8 illustrates the GPC-HPLC profile of cross-linked Hyaluronic acid with molecular weight of 1,000 Kd depolymerized with mammalian hyaluronidase after 7 hours of reaction. Signals of deca (14.17 min), octa (14.80 min), hexa (15.22 min) and tetrasaccharides (15.88 min) are indicated in the chromatogram.

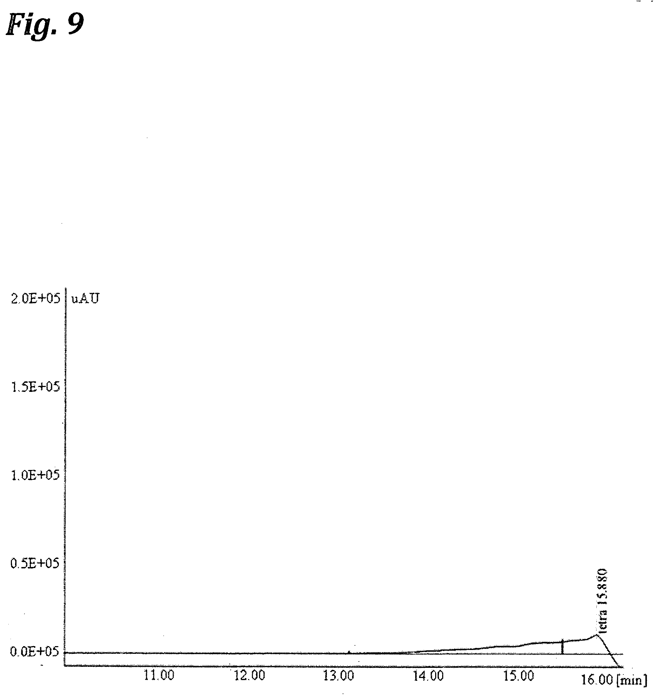

- Figure 9 illustrates the GPC-HPLC profile of cross-linked Hyaluronic acid with molecular weight of 100 Kd depolymerized with mammalian hyaluronidase after 7 hours of reaction. Signals of tetrasaccharides (15.88 min) are indicated in the chromatogram.

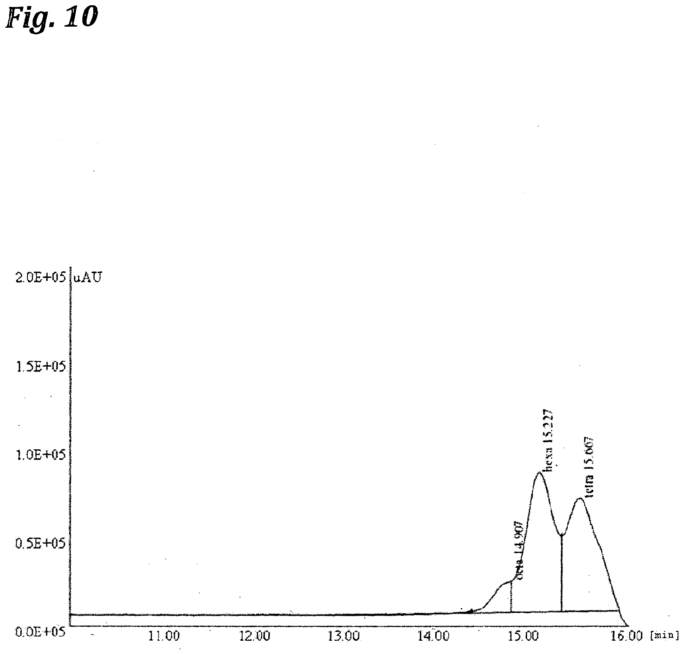

- Figure 10 illustrates the GPC-HPLC profile of Hyaluronic acid with molecular weight of 1,000 Kd depolymerized with mammalian hyaluronidase after 48 hours of reaction. Signals of octa (14.90 min), hexa (15.22 min) and tetrasaccharides (15.66 min) are indicated in the chromatogram.

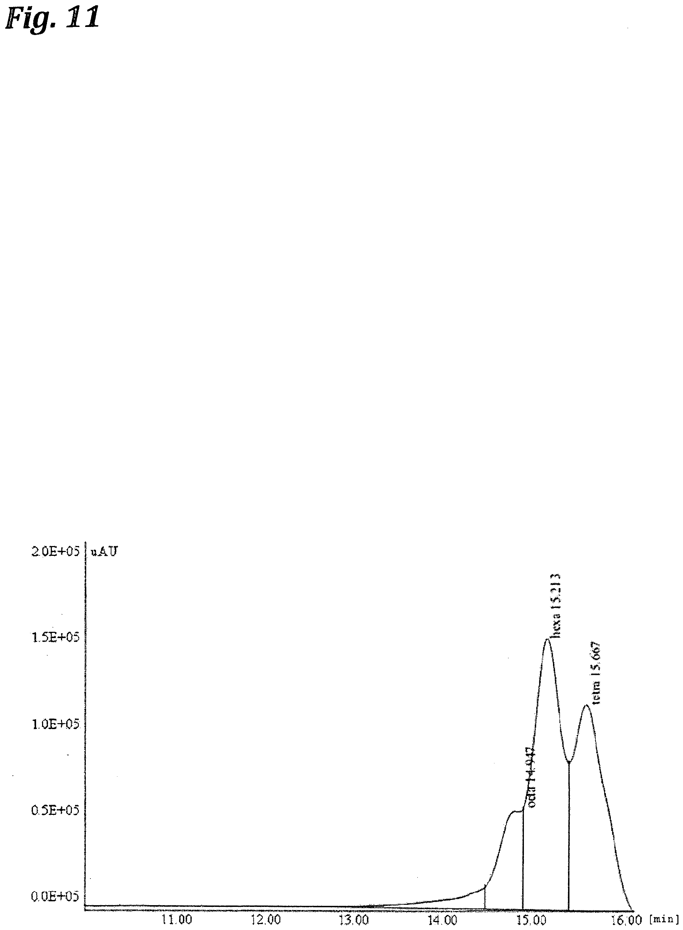

- Figure 11 illustrates the GPC-HPLC profile of a commercial cross-linked product (Durolane) depolymerized with mammalian hyaluronidase after 48 hours of reaction. Signals of octa (14.91 min), hexa (15.21 min) and tetrasaccharides 15.66 min) are indicated in the chromatogram.

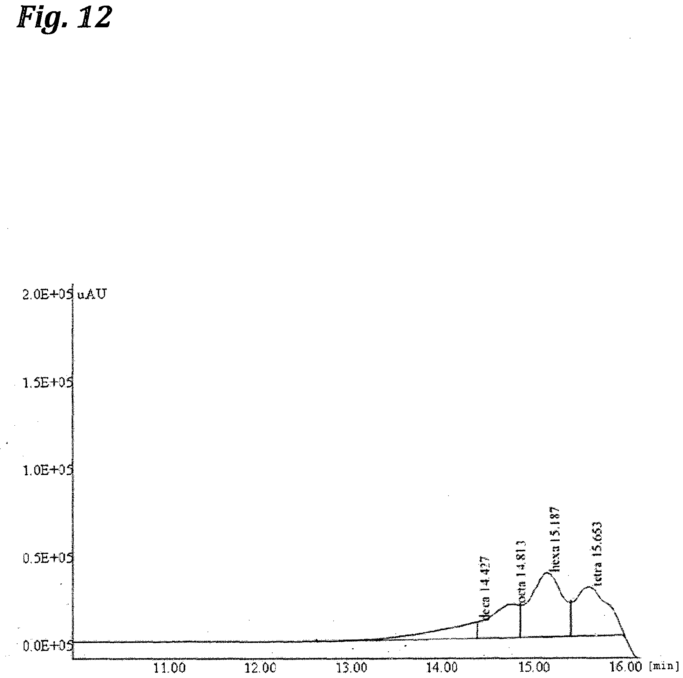

- Figure 12 illustrates the GPC-HPLC profile of cross-linked Hyaluronic acid with molecular weight of 1,000 Kd depolymerized with mammalian hyaluronidase after 48 hours of reaction. Signals of deca (14.42 min), octa (14.81 min), hexa (15.18 min) and tetrasaccharides(15.65 min) are indicated in the chromatogram.

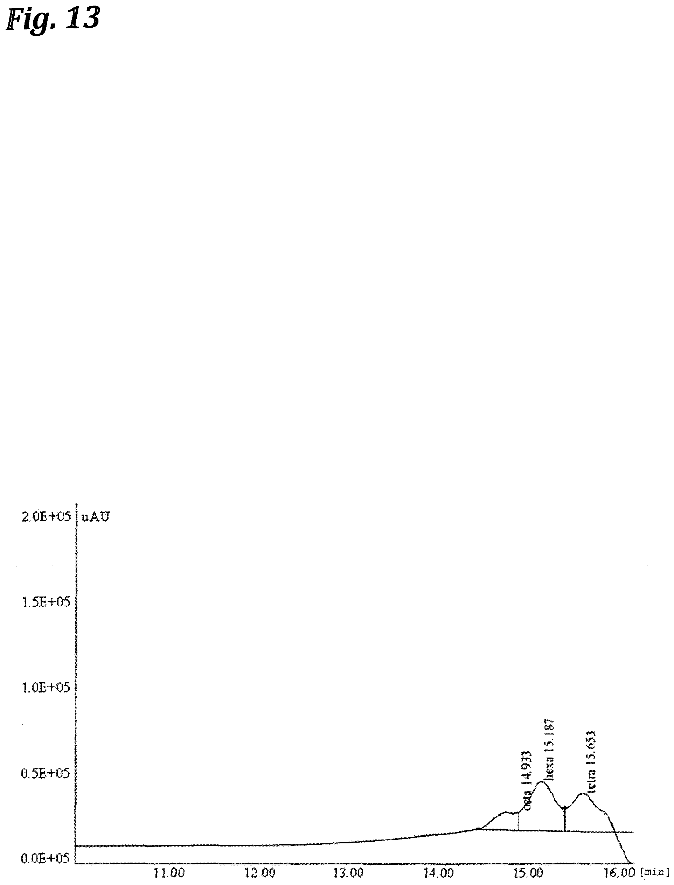

- Figure 13 illustrates the GPC-HPLC profile of cross-linked Hyaluronic acid with molecular weight of 100 Kd treated with mammalian hyaluronidase after 48 hours of reaction. Signals of octa (14.93 min), hexa (15.18 min) and tetrasaccharides (15.65 min) are indicated in the chromatogram.

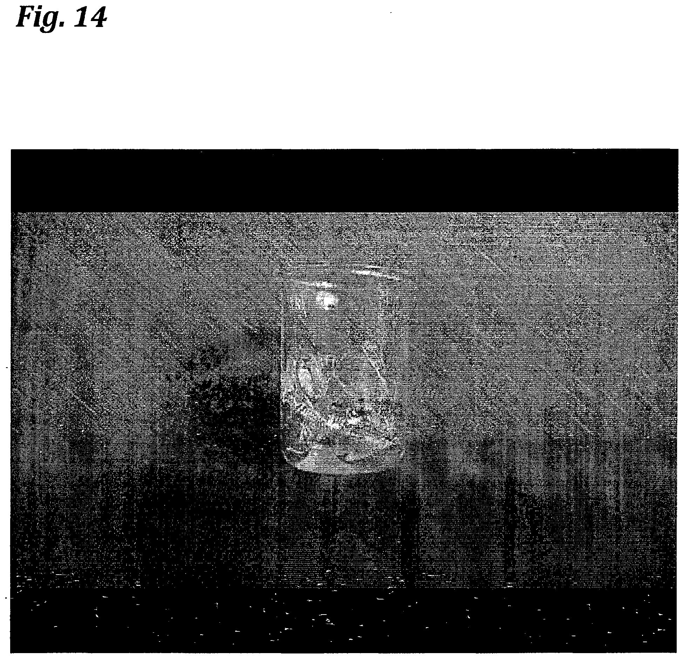

- Figure 14 illustrates the clear solid hydrogel of example 1

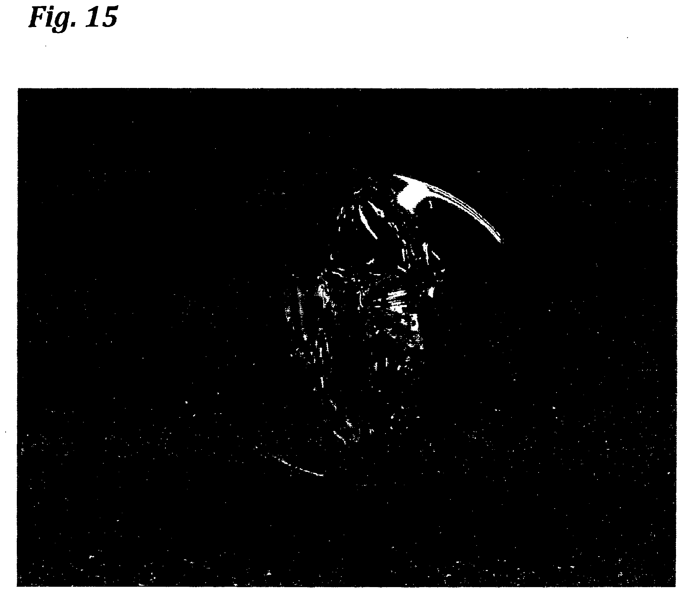

- Figure 15 illustrates the clear solid hydrogel of example 4

### DETAILED DESCRIPTION

Thus, the present invention provides a process for the preparation of a viscoelastic, solid and transparent cross-linked sodium hyaluronate hydrogel formed by a sodium hyaluronate substrate having a mean molecular weight of from 50,000 Da to 100,000 Da, cross-linked with BDDE.

The invention also provides the cross-linked, low-molecular-weight hyaluronic acid hydrogel obtained by the process.

The BDDE cross-linked low-molecular-weight sodium hyaluronates are prepared by reacting a sodium hyaluronate substrate having a mean molecular weight between 50,000 and 100,000 with 1,4-butanediol diglycidyl ether, in a molar ratio of from 1/180 to 1/500, in an aqueous solution of sodium hydroxide. The cross-linking reaction is carried out by reacting an 8%-40% solution of sodium hyaluronate with a mean molecular weight of from 50,000 to 100,000 in a 0.75%-1.5% aqueous sodium hydroxide solution with an amount of cross-linking agent of from 180 moles to 500 moles per mole of sodium hyaluronate.

Thus, it is an object of the present invention to provide a viscoelastic, solid and transparent, low-molecular-weight hyaluronate hydrogel obtainable by a process which comprises reacting an 8%-40% solution of a sodium hyaluronate having a mean molecular weight of from 50,000 to 100,000 Da in a 0.75%-1.5% aqueous sodium hydroxide solution with an amount of 1,4-butanediol diglycidyl ether of from 180 moles to 500 moles per mole of sodium hyaluronate.

According to a particular embodiment, the process affording the cross-linked hyaluronates of the present invention is carried out under the above conditions, starting from a sodium hyaluronate having a mean molecular weight of from 50,000 to 100,000 Da, at a temperature of from 45 to 60 °C for a period of time from 3 to 8 hours.

It is also an object of the present invention to provide a process for the preparation of viscoelastic, solid and transparent cross-linked low-molecular-weight sodium hyaluronate hydrogel, which comprises reacting an 8-40% solution of a sodium hyaluronate having a mean molecular weight of from 50,000 to 100,000 Da in a 0.75%-1.5% aqueous solution of sodium hydroxide with an amount of 1,4-butanediol diglycidyl ether corresponding to from 180 moles to 500 moles per mole of the sodium hyaluronate substrate at a temperature of from 45 to 60 °C for a period of time from 3 to 8 hours.

However, a viscoelastic, solid and transparent cross-linked HA (hyaluronate) hydrogel may also be obtained from a sodium hyaluronate substrate having a mean molecular weight of 100,000 Da by operating under a wider conditions' range, in particular by using a wider cross-linking agent/HA ratio range. In fact, such a hydrogel may also be obtained by reacting an 8-40% solution of a sodium hyaluronate having a mean molecular weight of 100,000 Da in a 0.75%-1.5% aqueous solution of sodium hydroxide with an amount of 1,4-butanediol diglycidyl ether corresponding to from 55 moles to 500 moles per mole of the sodium hyaluronate substrate at a temperature of from 42 to 60 °C for a period of time from 3 to 8 hours.

According to a preferred embodiment, a sodium hyaluronate having a mean molecular weight of 100,000 Da is dissolved at concentrations from 18% to 22% in a 0.75 - 1.25% sodium hydroxide solution, the obtained solution is added with 1,4-butanediol diglycidyl ether as cross-linking agent in an molar amount of from 55 to 60 moles of cross-linking agent per mole of hyaluronate and the solution is heated at 45-55°C for 4-6 hours.

At the end of the reaction, the BDDE cross-linked sodium hyaluronate hydrogel thus obtained is purified from unreacted reagents and from salts by known washing procedures, in particular by using phosphate buffer and water, and isolated as a semisolid, viscoelastic, transparent hydrogel.

According to another embodiment, a sodium hyaluronate having a mean molecular weight of 100,000 Da is dissolved at concentrations from 18% to 22% in a 0.75 - 1.25% sodium hydroxide solution, the obtained solution is added with 1,4-butanediol diglycidyl ether as the cross-linking agent in an molar amount of from 55 to 60 moles of cross-linking agent per mole of hyaluronate and the solution is heated at 42-55°C for 4-6 hours.

Thus, the present invention also provides a process for the preparation of a viscoelastic, semisolid and transparent cross-linked hyaluronate which comprises reacting a 18%-22% solution of a sodium hyaluronate having a mean molecular weight of 100,000 Da in a 0.75%-1.25% sodium hydroxide aqueous solution with 1,4-butanediol diglycidyl ether, in molar amount of from 55 moles to 65 moles per mole of sodium hyaluronate, at a temperature of 42-55 °C for a period of time from 4 to 6 hours.

The cross-linked HAs of the present invention may be used as viscosupplementaion fillers for treating joint diseases or as filler for tissue relaxation treatment (wrinkles). For such a use, the thus purified cross-linked, low-molecular-weight sodium hyaluronate hydrogel may be micronized, sterilized and presented as a unit form, such as a syringe.

Thus, the present invention further provides a viscosupplementation filler comprising a cross-linked sodium hyaluronate hydrogel obtainable by reacting a sodium hyaluronate having a mean molecular weight of 100,000 Da, dissolved at a concentration of from 18% to 22% in a 0.75 - 1.25% sodium hydroxide solution, with 1,4-butanediol diglycidyl ether (BDDE), in a molar amount of from 55 to 60 moles per mole of sodium hyaluronate, at 42-55 °C, preferably at 45-55°C for 4-6 hours, purified and optionally micronized and sterilized.

The use of the cross-linked HAs of the invention for use in biomedical, pharmaceutical and cosmetic applications constitutes another aspect of the invention.

The use of the cross-linked HA of the invention as viscosupplementation filler constitutes another aspect of the invention.

The following examples illustrate the invention.

### PREPARATION I (Comparative Example)

An amount of 1 g sodium hyaluronate powder with a mean molecular weight of 1,000 KDa (Shiseido) was dissolved in 4.5 ml of 1% Na OH (11% solution by weight in 0.25M NaOH). The solution was kept at room temperature for 4 hours under stirring and then cross-linked at 50°C for 5 hours in the presence of 60 µl butanediol diglycidyl ether (BDDE). The cross-linked sodium hyaluronate was left in 0.067 M Phosphate Buffer pH 7.4 and then washed three times with the same buffer and three times with water. A semisolid, clear hydrogel consisting of a cross-linked 1,000 KDa sodium hyaluronate was thus obtained.

### STANDARD PREPARATION A

### Solution of Native HA 1000 KDa

An amount of 10 mg sodium hyaluronate with mean molecular weight 1,000 KDa used as substrate in PREPARATION I was dissolved in 10 ml of 0.2 M Acetate Buffer pH 5.6 containing 140 mM NaCl and left to hydrate overnight at room temperature.

### STANDARD PREPARATION B

### Solution of Native HA 100 KDa

An amount of 10 mg sodium hyaluronate with mean molecular weight 100 KDa (Shiseido) was dissolved in 10 ml of 0.2 M Acetate Buffer pH 5.6 containing 140 mM NaCl and left to hydrate overnight at room temperature.

### STANDARD PREPARATION C

### Solution of Native HA 50 KDa

An amount of 10 mg sodium hyaluronate with mean molecular weight 50 KDa (Shiseido) was dissolved in 10 ml of 0.2 M Acetate Buffer pH 5.6 containing 140 mM NaCl and left to hydrate overnight at room temperature.

### SAMPLE PREPARATION D

### Solution of Cross-linked HA 1000 KDa

An amount of 10 mg of sample prepared as described in PREPARATION I was dissolved in 10 ml of 0.2 M Acetate Buffer pH 5.6 containing 140 mM NaCl.

### SAMPLE PREPARATION E

### Solution of Cross-linked HA 100 KDa

An amount of 10 mg of sample prepared as described in Example 1 below was dissolved in 10 ml of 0.2 M Acetate Buffer pH 5.6 containing 140 mM NaCl.

### SAMPLE PREPARATION F

### Solution of Cross-linked HA 100 KDa

An amount of 10 mg of sample prepared as described in Example 2 below was dissolved in 10 ml of 0.2 M Acetate Buffer pH 5.6 containing 140 mM NaCl.

### SAMPLE PREPARATION G

### Solution of Cross-linked HA 100 KDa

An amount of 10 mg of sample prepared as described in Example 3 below was dissolved in 10 ml of 0.2 M Acetate Buffer pH 5.6 containing 140 mM NaCl.

### SAMPLE PREPARATION H

### Solution of Cross-linked HA 50 KDa

An amount of 10 mg of sample prepared as described in Example 5 was dissolved in 10 ml of 0.2 M Acetate Buffer pH 5.6 containing 140 mM NaCl.

### SAMPLE PREPARATION J

### Solution of Cross-linked HA 50 KDa

An amount of 10 mg of sample prepared as described in Example 6 was dissolved in 10 ml of 0.2 M Acetate Buffer pH 5.6 containing 140 mM NaCl.

### PREPARATION K

### Solution of Commercial Cross-linked HA

An amount of 14 mg of a liquid commercial cross-linked HA was dissolved in 14 ml 0.2M Acetate Buffer pH 5.6 containing 140 mM NaCl.

### EXAMPLE 1

### Preparation of cross-linked NaHA from a 100 KDa NaHA substrate

An amount of 500 mg sodium hyaluronate in powder with a mean molecular weight of 100 KDa (Shiseido) was dissolved in 2.5 ml of 1% NaOH (20% solution by weight in 0.25M NaOH). The solution was kept at room temperature for 4 hours under stirring and then cross-linked at 50°C for 5 hours in the presence of 60 µl butanediol diglycidyl ether (BDDE), corresponding to 58 moles of BDDE per mole of HA-Na. The cross-linked hyaluronic acid was left in 0.067 M Phosphate Buffer pH 7.4 and then washed three times with the same buffer and three times with water to give a solid clear hydrogel consisting of a cross-linked 100 KDa sodium hyaluronate.

### EXAMPLE 2

### Preparation of cross-linked NaHA from a 100 KDa NaHA substrate

An amount of 1.1 g sodium hyaluronate in powder with a mean molecular weight of 100 KDa as that used in Example 1 was dissolved in 10 ml of 1% NaOH (11% solution by weight in 0.25M NaOH). The solution was kept at room temperature for 4 hours under stirring and then cross-linked at 50°C for 5 hours in the presence of 1 ml butanediol diglycidyl ether (BDDE), corresponding to 445 moles of BDDE per mole of HA-Na. The cross-linked hyaluronic acid was left in 0.067 M Phosphate Buffer pH 7.4 and then washed three times with the same buffer and three times with water to give a solid clear hydrogel consisting of a cross-linked 100 KDa sodium hyaluronate.

### EXAMPLE 3

### Preparation of cross-linked NaHA from a 100 KDa NaHA substrate

An amount of 400 mg sodium hyaluronate in powder with a mean molecular weight of 100 KDa as that used in Example 1 was dissolved in 1 ml of 1.3% NaOH (40% solution by weight ). The solution was kept at room temperature for 4 hours under stirring and then cross-linked at 50°C for 5 hours in the presence of 300 µl butanediol diglycidyl ether (BDDE), corresponding to 408 moles of BDDE per mole of HA-Na. The cross-linked hyaluronic acid was left in 0.067 M Phosphate Buffer pH 7.4 and then washed three times with the same buffer and three times with water to give a solid clear hydrogel consisting of a cross-linked 100 KDa sodium hyaluronate.

### EXAMPLE 4

An amount of 500 mg sodium hyaluronate in powder with a mean molecular weight of 100 KDa (Shiseido) was dissolved in 2.5 ml of 1% NaOH (20% solution by weight in 0.25M NaOH). The solution was kept at room temperature for 4 hours under stirring and then cross-linked at 42°C for 5 hours in the presence of 60 µl 1,4-butanediol diglycidyl ether (BDDE), corresponding to 58 moles of BDDE per mole of HA. The cross-linked hyaluronic acid was left in 0.067 M Phosphate Buffer pH 7.4 and then washed three times with the same buffer and three times with water to give a hydrogel consisting of a cross-linked 100 KDa sodium hyaluronate. A solid, clear hydrogel was obtained.

### EXAMPLE 5

### Preparation of cross-linked NaHA from a 50 KDa NaHA substrate

An amount of 1.1 g sodium hyaluronate in powder with a mean molecular weight of 50 KDa was dissolved in 10 ml of 1% NaOH (11% solution by weight). The solution was kept at room temperature for 4 hours under stirring and then cross-linked at 50°C for 4 hours in the presence of 1 ml butanediol diglycidyl ether (BDDE), corresponding to 222 moles of BDDE per mole of HA-Na. The cross-linked hyaluronic acid was left in 0.067 M Phosphate Buffer pH 7.4 and then washed three times with the same buffer and three times with water to give a solid clear hydrogel consisting of a cross-linked 50 KDa sodium hyaluronate.

### EXAMPLE 6

### Preparation of cross-linked NaHA from a 50 KDa NaHA substrate

An amount of 400 mg sodium hyaluronate in powder with a mean molecular weight of 50 KDa was dissolved in 1 ml of 1.3% NaOH (40% solution by weight ). The solution was kept at room temperature for 4 hours under stirring and then cross-linked at 50°C for 4 hours in the presence of 300 µl butanediol diglycidyl ether (BDDE), corresponding to 185 moles of BDDE per mole of HA-Na. The cross-linked hyaluronic acid was left in 0.067 M Phosphate Buffer pH 7.4 and then washed three times with the same buffer and three times with water to give a solid clear hydrogel consisting of a cross-linked 50 KDa sodium hyaluronate.

### EXAMPLE 7

### Method for Determining Enzyme Kinetics

A volume of 1 ml of each of the ten Preparations A-K (substrate) was poured into a vial in the dark. The solution was brought to 37°C in a water bath under slow stirring. Separately, a solution of hyaluronidase (enzyme solution) was prepared by dissolving the content of a vial of hyaluronidase from *Streptomyces hyalurolyticus* (SIGMA cat. N. H1136) in 1 ml of 0.2 M Acetate Buffer pH 5.6 containing 1 mg/ml of bovine serum albumin (BSA) as stabilizer. A volume of 200 µl of the enzyme solution thus obtained was added to the reaction vial of the substrate and 100 µl aliquots were taken at regular times and the reaction was stopped with the addition of 1 ml of 50mM KCl pH 1.8. After centrifugation at 3000 rpm for 20 minutes at 4°C, the absorbance of the solution was detected at 235 nm.

### EXAMPLE 8

### Enzyme Kinetics Comparison

The absorbances of the hyaluronic acid of the Standard Preparation A and of the cross-linked Sample Preparation D, determined according to the method of Example 7 at the different times, are reported in Table 1.

**Table 1**

| **Hours** | **Standard HA 1000 KDa (A)** | **Cross-linked 1000 KDa (D)** |
|---|---|---|
| 0 | 0,0 | 0 |
| 1 | 230,9 | 40,9 |
| 2 | 290,0 | 52,6 |
| 3 | 291,3 | 53,7 |
| 18 | 306,5 | 91,2 |
| 20 | 310,1 | 93,4 |
| 23 | 313,4 | 99,8 |

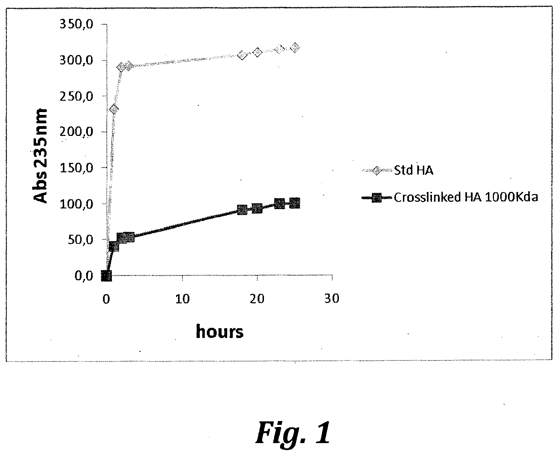

The degradation kinetics by hyaluronidase of the two products of Table 1 is given in Figure 1. This comparison shows that, at the 23^{rd} hour, the degradation of the cross-linked HA 1000 KDa is about three times lower than that of the standard HA 1000 KDa

### EXAMPLE 9

### Enzyme Kinetics Comparison

The absorbances of the hyaluronic acid of the Standard Preparation A, of the Standard Preparation B and of the cross-linked Sample Preparation E of the present invention, determined according to the method of Example 7 at the different times, are reported in Table 2.

**Table 2**

| **Hours** | **Standard HA 1000 KDa (A)** | **Standard HA 100 KDa (B)** | **Cross-linked 100 KDa (E)** |
|---|---|---|---|
| 0 | 0,0 | 0 | 0 |
| 1 | 230.9 | 138.8 | 16.2 |
| 2 | 290.0 | 181.6 | 17.7 |
| 3 | 296.1 | 189.2 | 18.0 |
| 23 | 313.4 | 200.5 | 23.4 |
| 25 | 316.5 | 201.3 | 22.4 |

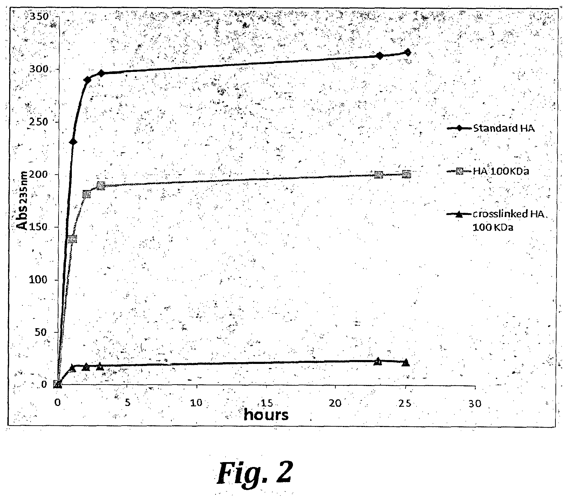

The degradation kinetics by hyaluronidase of the three products of Table 2 is given in Figure 2. This comparison shows that, at the 25th hour, the degradation of the cross-linked HA 100 KDa of example 1 of the present invention is about fourteen times lower than that of the standard HA 1000 Kda and about nine times lower than that of the standard HA 100 KDa

### EXAMPLE 10

### Enzyme Kinetics Comparison

The absorbances of the commercial, cross-linked hyaluronic acid of Preparation K and of the cross-linked Sample Preparation E of the present invention, determined according to the method of Example 7 at the different times, are reported in Table 3.

**Table 3**

| **Hours** | **Commercial Cross-linked HA (K)** | **Cross-linked HA 100 KDa (E)** |
|---|---|---|
| 0 | 0 | 0 |
| 1 | 24.8 | 4.7 |
| 2 | 30.0 | 8.1 |
| 3 | 35.6 | 11.1 |
| 4 | 37.7 | 13.0 |
| 24 | 39.9 | 13.5 |
| 26 | 40.2 | 13.7 |
| 28 | 40.4 | 13.4 |

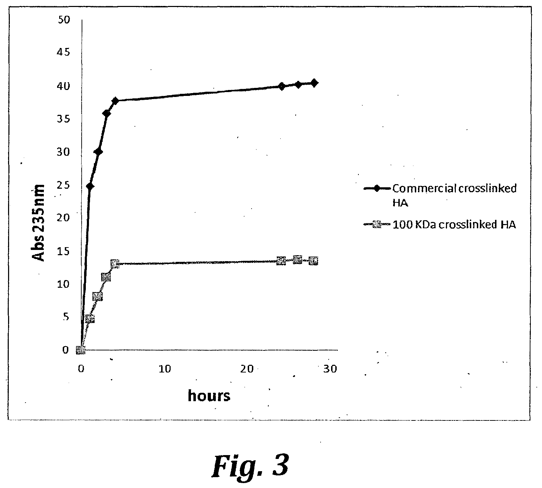

The degradation kinetics by hyaluronidase of the two products of Table 3 is given in Figure 3. This comparison shows that, at the 28th hour, the degradation of the cross-linked HA 100 KDa of example 1 of the present invention is about three times lower than that of the commercial HA 1000 KDa

### EXAMPLE 11

### Enzyme Kinetics Comparison

The absorbances of the native HA 100 KDa of Preparation B and of the cross-linked Sample Preparations E, F, G of the present invention, determined according to the method of Example 7 at the different times, are reported in Table 4.

**Table 4**

| **hours** | **HA 100K(B)** | **100KDa 11%(F)** | **100KDa 40%(G)** | **100KDa 20%(E)** |
|---|---|---|---|---|
| 0 | 0 | 0 | 0 | 0 |
| 1 | 164,9 | 19,2 | 25,4 | 9,2 |
| 2 | 197,2 | 23,4 | 27,1 | 15,9 |
| 3 | 223,3 | 24,9 | 29,1 | 22 |
| 4 | 240,4 | 28,3 | 35,4 | 24,5 |
| 24 | 242 | 28,7 | 36 | 26,9 |
| 26 | 243,2 | 28,3 | 38,1 | 28,5 |
| 28 | 241,9 | 27,8 | 37 | 29,2 |

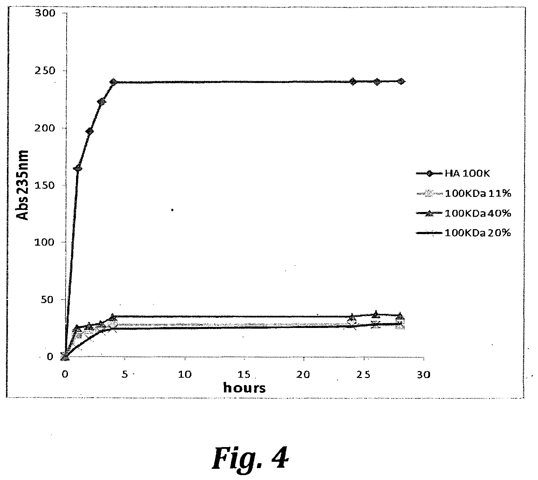

The degradation kinetics by hyaluronidase of the four products of Table 4 is given in Figure 4. This comparison shows that, at the 28th hour, the degradation kinetics of the cross-linked HA 100 KDa of Examples 1, 2 and 3 of the present invention are from 6 to 8 times lower than that of the 100 KDa native material of PREPARATION B.

### EXAMPLE 12

### Enzyme Kinetics Comparison

The absorbances of the native HA 50 KDa of Preparation C and of the cross-linked Sample Preparations H and J of the present invention, determined according to the method of Example 7 at the different times, are reported in Table 5.

**Table 5**

| hours | **HA 50KDa STD(C)** | **50KDa 11%(H)** | **50KDa 40%(J)** | **100KDa 20%(E)** |
|---|---|---|---|---|
| 0 | 0 | 0 | 0 | 0 |
| 1 | 88,6 | 9 | 19,7 | 10,7 |
| 2 | 120,3 | 10,2 | 20,6 | 12,9 |
| 3 | 133,2 | 13,4 | 21,5 | 17,1 |
| 4 | 145,2 | 17,5 | 24,4 | 20,1 |
| 24 | 145,3 | 17,5 | 33,3 | 26,8 |
| 26 | 145,8 | 17,7 | 34,8 | 27,5 |
| 28 | 145,6 | 17,7 | 34,5 | 28 |
| 30 | 146 | 17,8 | 34,7 | 29,2 |

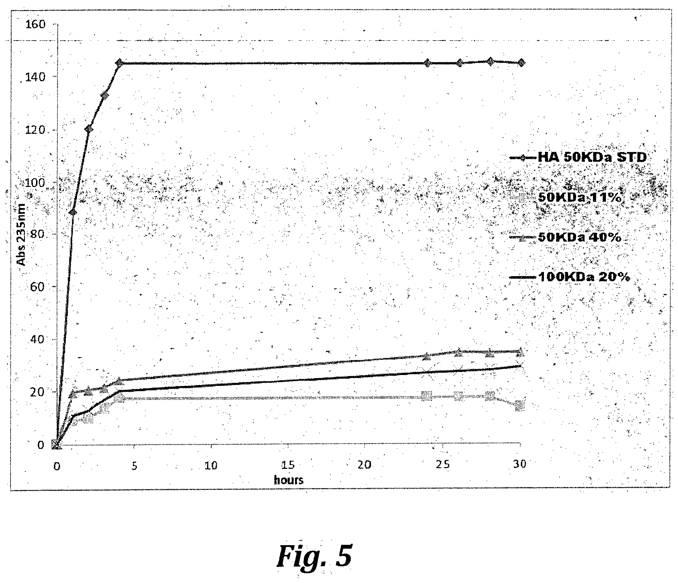

The degradation kinetics by hyaluronidase of the four products of Table 5 is given in Figure 5. This comparison shows that, at the 30th hour, the degradation kinetics of the cross-linked HA 50 KDa of examples 5 and 6 of the present invention are from 4 to 8 times lower than that of the 50KDa starting material of PREPARATION C. They are also of the same resistance or half time more resistant to the enzymic depolymerization than the 100KDa cross-linked sample of example 1.

### EXAMPLE 13

### Depolymerization with mammalian hyaluronidase

To investigate in vitro the resistance to the enzymes of the hydrogel in the pathological situation of arthrosic patients in which there is a overproduction of hyaluronidase, the semisolid hydrogel of example 1 was depolymerized in presence of excess of mammalian hyaluronidase in comparison with a commercial crosslinked hyaluronic acid, a 1,000,000 Da Hyaluronic acid and a 1,000,000 Da crosslinked hyaluronic acid synthesised with the same reaction of the product of example 1 and the reaction products were analysed by GPC-HPLC.

An amount of 6 mg of sample were dissolved in 2 ml of 0.3 M phosphate buffer pH 5.5 and left overnight at room temperature. Then the solution was diluted 1:10 with the same buffer and 0.5 ml of 20mM phosphate buffer containing 77mM NaCl pH 7.0 were added to 2ml of sample to be depolymerized. The solution was kept at 37°C.

An amount of 10 mg of hyaluronidase type IV from bovine testis (SIGMA-ALDRICH) were dissolved in 14.92 ml of 20 mM phosphate buffer containing 77mM NaCl pH 7.0 and diluted 1:25 with the same buffer to have 40 U/ml. The enzyme solution was kept at 37°C for 10 minutes. Then 1.5 ml of enzyme solution was added to the sample solution at 37°C and left to react at for 7 hours. A volume of 1 ml of reaction mixture was withdrawn and the reaction was stopped by boiling at 100°C. Before the HPLC analysis, the sample was filtered with a 0.2 µm membrane.

After 8 hours of reaction 750 µl of enzyme were added to each sample and the reaction was continued for further 40 hours. At the end, the reaction was stopped by boiling at 100°C, filtered with a 0.2 µm membrane and analyzed by HPLC.

The assay was performed by a HPLC instrument with auto-injector, pump and UV detector, with a GPC column (Cosmosil Diol 120 II 5 um 7.5 x 300 mm with precolumn from Nacalai - USA) and 0.15 M sodium sulfate buffer as isocratic mobile phase with a flux of 0.6 ml/min. Samples were filtrated through a 0.45 µm filter and 100 µl of solution was injected in the HPLC system. The signals are detected at 205 nm along all the total run time (25 minutes). The retention times (RT) of the signals were compared with the RT of calibrated oligosaccharide standards with the following RT ranges :
Dodecasaccharide : 13.80-14.05 min
Decasaccharide : 14.05-14.45 min
Octasaccharide : 14.70-15.00 min
Hexasaccharide : 15.05-15.25 min
Tetrasaccharide : 15.50-15.90 min

### EXAMPLE 14

### (Biological Activity)

Two samples of cross-linked hyaluronic acid, a commercial sample (Durolane®, a cross-linked, high-molecular-weight Na-HA) and the hydrogel of Example 1 (GL 2570), have been tested on chondrocytes. Chondrocytes were collected from 3 patients with osteoarthritis, therefore with an overproduction of HA degradation enzymes (hyaluronidases). Increasing amounts of samples (200, 500 and 1000 µg/ml) were added to the cell cultures with different cell concentrations (12,000 and 22,500 cells) and tested at different times (24, 48 and 72 hours). In the first analysis the morphology of the cells after the addition of the samples was analyzed by microscope. The shape of the cells appeared unchanged at all the concentrations. Then alive and dead cells have been tested with the LIVE/DEAD Viability/Cytotoxicity KIT cat. n° L3224 (Invitrogen), in which alive cells have a green color while dead cells are colored in red. In both cases (Durolane and GL 2570) no red cells were detected in all the concentrations at all the times. The compound of Example 1 (GL 2570) does not induce cellular death.

To test the cellular metabolism correlated to the cell proliferation, the MTT test was applied (MTT cat. n° M-5655,SIGMA). No differences were detected after the addition of the sample to the chondrocytes at all the concentrations ad times.

To evaluate the cellular toxicity of the products, the LDH formation was measured by Cytotoxicity Detection KIT (LDH) cat. n° 11644793001 (ROCHE). LDH is an index of the cell membrane integrity and is overproduced in the presence of a toxic agent. In all collected chondrocyte samples no toxicity was detected for both the samples.

The proliferation was tested by measuring the incorporation of tritiated thymidine. Thymidine is incorporated by cells to synthesize new DNA for the new generation of cells. In this test the two samples appeared different. At least in one patient the incorporation of tritiated thymidine by cells treated with Durolane increases at 24 hours while in cells treated with GL 2570 the tritiated thymidine is not incorporated up to 48 hours and then slightly increases at 72 hours, but without reaching the Durolane value. This difference is correlated to the resistance to hyaluronidase action. In particular, within the first 24 hours GL 2570 is not depolymerized by hyaluronidase and the layer of the cross-linked compound blocks the incorporation of various biological and chemical agents, including tritiated Thymidine. Durolane on the contrary is immediately degraded and cannot inhibit the permeation of substances into cells.

## Claims

1. A process for the preparation of a viscoelastic, solid and transparent cross-linked low-molecular-weight sodium hyaluronate hydrogel, which comprises reacting an 8-40% solution of a sodium hyaluronate substrate having a mean molecular weight of from 50,000 Da to 100,000 Da in a 0.75%-1.5% aqueous solution of sodium hydroxide with an amount of 1,4-butanediol diglycidyl ether corresponding to from 180 moles to 500 moles per mole of the sodium hyaluronate substrate at a temperature of from 45 to 60 °C for a period of time from 3 to 8 hours.

2. A process for the preparation of a viscoelastic, solid and transparent cross-linked low-molecular-weight sodium hyaluronate hydrogel, which comprises reacting an 8-40% solution of a sodium hyaluronate substrate having a mean molecular weight of 100,000 Da in a 0.75%-1.5% aqueous solution of sodium hydroxide with an amount of 1,4-butanediol diglycidyl ether corresponding to from 55 moles to 500 moles per mole of said substrate at a temperature of from 42°C to 60°C for a period of time from 3 to 8 hours.

3. The process of claim 2, wherein the amount of 1,4-butanediol diglycidyl ether corresponds to from 55 moles to 60 moles per mole of said substrate and the reaction is carried out at 45-55 °C for 4-6 hours.

4. The process of any one of claims 1 to 3, **characterized in that** said process also comprises a final washing procedure using phosphate buffer and water.

5. The process of claim 1, **characterized in that** the starting sodium hyaluronate has a mean molecular weight of 50,000 Da.

6. The process of claim 5, which comprises reacting an 11% aqueous solution of a sodium hyaluronate having a mean molecular weight of 50,000 Da in a 1% aqueous solution of sodium hydroxide with an amount of 1,4-butanediol diglycidyl ether corresponding to 222 moles per mole of the sodium hyaluronate substrate at 50°C for 4 hours.

7. A viscoelastic, solid and transparent cross-linked low-molecular-weight sodium hyaluronate hydrogel, obtained by the process of any one of claims 1 to 6.

8. The sodium hyaluronate hydrogel of claim 7, for use in biomedical, pharmaceutical and cosmetic applications.

9. The sodium hyaluronate hydrogel for use according to claim 8, as a viscosupplementation filler.

## Patentansprüche

1. Ein Verfahren zur Herstellung eines viskoelastischen, festen und transparenten vernetzten niedermolekularen Natriumhyaluronat-Hydrogels, das die Reaktion einer 8-40%igen Lösung eines Natriumhyaluronat-Substrats mit einem mittleren Molekulargewicht von 50.000 Da bis 100.000 Da in einer 0,75-1,5%igen wässrigen Lösung von Natriumhydroxid mit einer Menge an 1,4-Butandioldiglycidylether, entsprechend von 180 Mol bis 500 Mol pro Mol des Natriumhyaluronat-Substrats, bei einer Temperatur von 45 bis 60 °C für einen Zeitraum von 3 bis 8 Stunden umfasst.

2. Ein Verfahren zur Herstellung eines viskoelastischen, festen und transparenten vernetzten niedermolekularen Natriumhyaluronat-Hydrogels, das die Reaktion einer 8-40%igen Lösung eines Natriumhyaluronat-Substrats mit einem mittleren Molekulargewicht von 100.000 Da in einer 0,75-1,5%igen wässrigen Lösung von Natriumhydroxid mit einer Menge an 1,4-Butandioldiglycidylether, entsprechend von 55 Mol bis 500 Mol pro Mol des Substrats, bei einer Temperatur von 42 °C bis 60 °C für einen Zeitraum von 3 bis 8 Stunden umfasst.

3. Das Verfahren gemäß Anspruch 2, wobei die Menge an 1,4-Butandioldiglycidylether 55 Mol bis 60 Mol pro Mol des Substrats entspricht und die Reaktion bei 45 bis 55 °C für einen Zeitraum von 4 bis 6 Stunden durchgeführt wird.

4. Das Verfahren gemäß irgendeinem der Ansprüche 1 bis 3, **dadurch gekennzeichnet, dass** das Verfahren auch ein abschließendes Waschverfahren unter Verwendung von Phosphatpuffer und Wasser umfasst.

5. Das Verfahren gemäß Anspruch 1, **dadurch gekennzeichnet, dass** das Ausgangssubstrat Natriumhyaluronat ein mittleres Molekulargewicht von 50.000 Da aufweist.

6. Das Verfahren gemäß Anspruch 5, das die Reaktion einer 11 %igen wässrigen Lösung eines Natriumhyaluronats mit einem mittleren Molekulargewicht von 50.000 Da in einer 1%igen wässrigen Lösung von Natriumhydroxid mit einer Menge an 1,4-Butandioldiglycidylether, entsprechend 222 Mol pro Mol des Natriumhyaluronat-Substrats, bei einer Temperatur von 50 °C für 4 Stunden umfasst.

7. Ein viskoelastisches, festes und transparentes vernetztes niedermolekulares Natriumhyaluronat-Hydrogel, erhalten durch das Verfahren gemäß irgendeinem der Ansprüche 1 bis 6.

8. Das Natriumhyaluronat-Hydrogel gemäß Anspruch 7, zur Verwendung in biomedizinischen, pharmazeutischen und kosmetischen Anwendungen.

9. Das Natriumhyaluronat-Hydrogel zur Verwendung gemäß Anspruch 8 als ein viskositätsergänzender Füllstoff.

## Revendications

1. Procédé de préparation d'un hydrogel hyaluronate de sodium de faible masse moléculaire réticulé, viscoélastique, solide et transparent, qui comprend la réaction d'une solution de 8 à 40 % d'un substrat hyaluronate de sodium ayant un masse moléculaire moyen de 50 000 Da à 100 000 Da dans une solution aqueuse d'hydroxyde de sodium de 0,75 % à 1,5 % avec une quantité d'éther de 1,4-butanediol diglycidyle correspondant à 180 moles à 500 moles par mole de substrat hyaluronate de sodium à une température de 45 à 60 °C pendant une période de temps de 3 à 8 heures.

2. Procédé de préparation d'un hydrogel hyaluronate de sodium de faible masse moléculaire réticulé, viscoélastique, solide et transparent, qui comprend la réaction d'une solution de 8 à 40 % d'un substrat hyaluronate de sodium ayant un masse moléculaire moyen de 100 000 Da dans une solution aqueuse de 0,75 % à 1,5 % d'hydroxyde de sodium avec une quantité d'éther de 1,4-butanediol diglycidyle correspondant de 55 moles à 500 moles par mole dudit substrat à une température de 42 °C à 60 °C pendant une période de temps de 3 à 8 heures.

3. Procédé selon la revendication 2, dans lequel la quantité d'éther de 1,4-butanediol diglycidyle correspond de 55 moles à 60 moles par mole dudit substrat et la réaction est effectuée de 45 à 55 °C pendant 4 à 6 heures.

4. Procédé selon l'une quelconque des revendications 1 à 3, **caractérisé en ce que** ledit procédé comprend également une procédure de lavage final utilisant un tampon phosphate et de l'eau.

5. Procédé selon la revendication 1, **caractérisé en ce que** le hyaluronate de sodium de départ a un masse moléculaire moyen de 50 000 Da.

6. Procédé selon la revendication 5, qui comprend la réaction d'une solution aqueuse à 11 % d'un hyaluronate de sodium ayant un masse moléculaire moyen de 50 000 Da dans une solution aqueuse à 1% d'hydroxyde de sodium avec une quantité d'éther de 1,4-butanediol diglycidyle correspondant à 222 moles par mole du substrat hyaluronate de sodium à 50 °C pendant 4 heures.

7. Hydrogel hyaluronate de sodium réticulé de faible masse moléculaire, viscoélastique, solide et transparent, obtenu par le procédé selon l'une quelconque des revendications 1 à 6.

8. Hydrogel hyaluronate de sodium selon la revendication 7, destiné à être utilisé dans des applications biomédicales, pharmaceutiques et cosmétiques.

9. Hydrogel hyaluronate de sodium selon la revendication 8, destiné à être utilisé comme charge de viscosupplémentation.
